(19) Europäisches Patentamt
European Patent Office
Office européen des brevets

(11) Veröffentlichungsnummer: **0 051 803**
**B1**

(12) # EUROPÄISCHE PATENTSCHRIFT

(45) Veröffentlichungstag der Patentschrift: **04.04.84**

(51) Int. Cl.³: **C 07 C 85/11**, C 07 C 87/56

(21) Anmeldenummer: **81109098.4**

(22) Anmeldetag: **28.10.81**

(54) **Verfahren zur Herstellung von 3,5-Dimethylanilin (sym. m-Xylidin).**

(30) Priorität: **06.11.80 DE 3041848**

(43) Veröffentlichungstag der Anmeldung: **19.05.82 Patentblatt 82/20**

(45) Bekanntmachung des Hinweises auf die Patenterteilung: **04.04.84 Patentblatt 84/14**

(84) Benannte Vertragsstaaten: **CH DE FR GB LI**

(56) Entgegenhaltungen:
**DE - B - 2 654 852**
**US - A - 3 956 389**

**HOUBEN-WEYL "Methoden der organischen Chemie" 4.Auflage, Band IV/2 1955,GEORG THIEME VERLAG, Stuttgart Seite 326, letzter Absatz**

(73) Patentinhaber: **HOECHST AKTIENGESELLSCHAFT, Postfach 80 03 20, D-6230 Frankfurt am Main 80 (DE)**

(72) Erfinder: **Müller, Werner Heinrich, Dr., Taunusblick 5, D-6239 Eppstein/Taunus (DE)**
Erfinder: **Berthold, Rüdiger, Dr., Geierfeld 55, D-6232 Bad Soden am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 803 B1

BUNDESDRUCKEREI BERLIN

Verfahren zur Herstellung von 3,5-Dimethylanilin (sym. m-Xylidin)

Gegenstand der vorliegenden Erfindung ist ein Verfahren zur Herstellung von 3,5-Dimethylanilin durch katalytische Dehydrierung von 3,5-Dimethylcyclohexenonoxim in der Gasphase.

3,5-Dimethylanilin ist ein Vorprodukt zur Herstellung von Farbstoffen und kann sowohl als Diazokomponente bei Azofarbstoffen als auch als Kondensationskomponente für Pigmentfarbstoffe der Perylentetracarbonsäurereihe verwendet werden.

Bisher stellte man 3,5-Dimethylanilin durch Umsetzung von sym. m-Xylenol mit Ammoniak bei hohen Temperaturen und Drücken her. Dieses Verfahren bedingt einen sehr großen technischen und apparativen Aufwand und benötigt als Vorprodukt das aus Steinkohlenteer gewonnene sym. m-Xylenol. Außerdem ist dieses Xylenol nicht gänzlich isomerenfrei.

Ziel der vorliegenden Erfindung war es daher, eine vom sym. m-Xylenol unabhängige Herstellung des 3,5-Dimethylanilins zu finden.

Es ist bekannt (Krauch, Kunz: Reaktionen der organischen Chemie, 5. Auflage, 1976, Seite 585), daß man Oxime bestimmter alicyclischer Ketone mittels Acetanhydrid und Schwefelsäure der Salzsäure in die entsprechenden Acetanilide überführen kann. Aus diesen lassen sich durch Verseifung mittels Alkalilaugen die gewünschten aromatischen Amine erhalten. Diese Methode ist jedoch sehr umständlich und bedeutet wegen der zwangsläufig gebildeten Alkalisalze eine erhebliche Umweltbelastung.

Versuche von E. C. Horning et al. (J. Am. Chem. Soc. 69, 1907 [1947]), 3,5-Dimethylcyclohexenonoxim katalytisch mittels Palladium/Kohle in Triethylbenzol als Lösemittel in 3,5-Dimethylanilin zu überführen, waren erfolglos.

Es wurde nun ein Verfahren zur Herstellung von 3,5-Dimethylanilin gefunden, das dadurch gekennzeichnet ist, daß man 3,5-Dimethylcyclohexenonoxim in der Gasphase bei Temperaturen von 200 bis 500°C über einen mindestens ein Edelmetall der 8. Nebengruppe des Periodensystems enthaltenden Katalysator leitet.

3,5-Dimethylcyclohexenonoxim kann auf folgende Weise (DE-OS 2 654 850; Ann. 281, 104 [1894]) hergestellt werden:

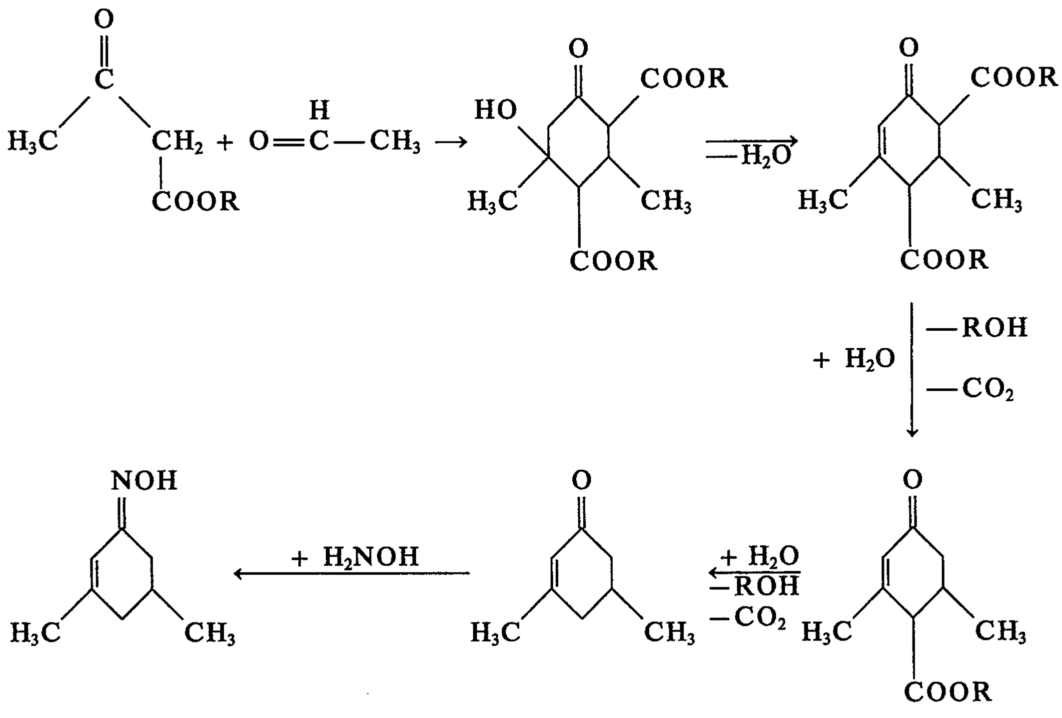


Zur erfindungsgemäßen Umsetzung des 3,5-Dimethylcyclohexenonoxims verwendet man im allgemeinen Katalysatoren, die Ruthenium, Rhodium, Palladium, Iridium oder Platin oder deren Gemische enthalten. Palladium, Platin und Palladium/Platin sind bevorzugt.

Im allgemeinen werden geträgerte Katalysatoren, beispielsweise mit Kohle, $SiO_2$, $Al_2O_3$, Aluminiumsilikaten, Spinellen, Chromoxid-Aluminiumoxid und Zeolithen als Trägern, verwendet. Die Konzentration des Edelmetalls auf dem Träger beträgt dabei im allgemeinen 0,05 bis 10 Gew.-%, vorzugsweise 0,2 bis 5 Gew.-%, insbesondere 0,3 bis 2,5 Gew.-%, jeweils bezogen auf das Gewicht des Trägers. Die Edelmetalle werden als Verbindungen auf den Träger aufgebracht und vorzugsweise vor Beginn der Reaktion reduziert, z. B. durch Überleiten von Wasserstoff.

Der Katalysator kann entweder als Festbett, als bewegtes Bett oder als Wirbelschicht angeordnet

sein.
Die Reaktionstemperatur beträgt im allgemeinen 200–500° C, vorzugsweise 250–400° C, insbesondere 270–380° C.
Im allgemeinen wird Unterdruck bis herab zu etwa 10 mbar oder Normaldruck angewandt, man kann jedoch auch bei höheren Drucken bis etwa 20 bar arbeiten. Vorzugsweise verwendet man ein Trägergas, um das Oxim über den Katalysator zu transportieren. Als Trägergase eignen sich besonders Wasserstoff, Stickstoff, $NH_3$, Argon, $CO_2$, Methan, Wasserdampf, Ethylen und Propylen.
Das Oxim kann vor dem Reaktor auch mit leichtflüchtigen Lösemitteln, wie z. B. Kohlenwasserstoffen, Ethern, insbesondere Glykol- und Polyglykol-dialkylethern, oder Wasser verdünnt werden.
Das folgende Beispiel dient zur Erläuterung des erfindungsgemäßen Verfahrens. Die angegebenen Litermengen an $N_2$ und $H_2$ beziehen sich auf den Normzustand (0° C, 1,013 bar).

### Beispiel

In einem Glasreaktor von 18 mm Durchmesser und 40 cm Länge befanden sich von oben nach unten zuerst eine 5 cm lange Schicht aus Glaskugeln und unmittelbar danach eine 16 cm lange Schicht eines Katalysators, bestehend aus 1 Gew.-% Pd auf $SiO_2$-Kugeln. Der Reaktor wurde im $N_2$-Strom auf 300° C aufgeheizt, anschließend mit 24 l Stickstoff und 6 l Wasserstoff pro Stunde 2 Stunden lang bei 280° C aktiviert.
Daraufhin wurden in 4 Stunden 200 g einer Mischung, bestehend aus 162 g Dimethyldiglykol und 38 g 3,5-Dimethylcyclohexen-2-onoxim-1 zusammen mit 80 l Stickstoff und 56 l Wasserstoff von oben über den durch eine elektrische Heizung auf 280° C erhitzten Katalysator gegeben.
Am Reaktorausgang wurden 184,4 g Produkt kondensiert. Die gaschromatographische Analyse ergab 26,1 g 3,5-Dimethylanilin, d. h. 79% d. Th., und 3,5 g Dixylylamin = 11,4% d. Th.

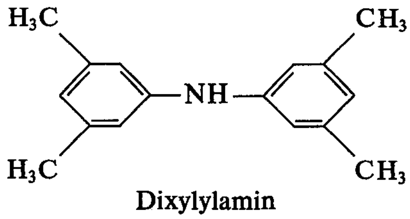

Dixylylamin

Nach Abdestillieren des Dimethyldiglykols ließ sich durch Vakuumdestillation reines 3,5-Dimethylanilin gewinnen.

**Patentansprüche**

1. Verfahren zur Herstellung von 3,5-Dimethylanilin, dadurch gekennzeichnet, daß man 3,5-Dimethylcyclohexenonoxim in der Gasphase bei Temperaturen von 200 bis 500° C über einen mindestens ein Edelmetall der 8. Nebengruppe des Periodensystems enthaltenden Katalysator leitet.
2. Verfahren nach Anspruch 1, dadurch gekennzeichnet, daß man als Edelmetall Palladium oder Platin oder deren Gemisch einsetzt.
3. Verfahren nach Anspruch 1 oder 2, dadurch gekennzeichnet, daß man bei Temperaturen von 270 bis 380° C arbeitet.

**Claims**

1. A process for the preparation of 3,5-dimethylaniline characterized by passing 3,5-dimethyl-cyclohexenone oxime in the gaseous phase at temperatures of from 200 to 500° C over a catalyst containing at least one noble metal of the 8th subgroup of the Periodic Table.
2. The process as claimed in Claim 1, characterized by using palladium or platinum or mixtures thereof as noble metal.
3. The process as claimed in Claim 1 or 2, characterized by operating at temperatures of from 270 to 380° C.

**Revendications**

1. Procédé de préparation de la 3,5-diméthylaniline, caractérisé en ce que l'on fait passer en phase gazeuse l'oxime de la 3,5-diméthylcyclohexénone, à des températures de 200 à 500° C, sur un catalyseur comprenant un ou plusieurs métaux nobles du sous-groupe 8 de la classification périodique

des éléments.

2. Procédé selon la revendication 1 caractérisé en ce que l'on utilise comme métal noble du palladium ou du platine ou un mélange des deux.

3. Procédé selon la revendication 1 ou 2, caractérisé en ce que l'on opère à des températures de 270 à 380° C.